(19) Europäisches Patentamt
European Patent Office
Office européen des brevets

(11) EP 2 671 578 A1

(12) **EUROPEAN PATENT APPLICATION**
published in accordance with Art. 153(4) EPC

(43) Date of publication:
**11.12.2013 Bulletin 2013/50**

(21) Application number: **12742127.9**

(22) Date of filing: **30.01.2012**

(51) Int Cl.:
***A61K 31/201*** *(2006.01)* ***A61P 25/02*** *(2006.01)*

(86) International application number:
**PCT/JP2012/051938**

(87) International publication number:
**WO 2012/105476 (09.08.2012 Gazette 2012/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2011 JP 2011018924**

(71) Applicants:
- **Nippon Zoki Pharmaceutical Co., Ltd.**
  **Osaka-shi**
  **Osaka 541-0046 (JP)**
- **Nagoya Industrial Science Research Institute**
  **Nagoya-shi**
  **Aichi 460-0008 (JP)**

(72) Inventors:
- **IINUMA, Munekazu**
  **Gifu-shi**
  **Gifu 501-1196 (JP)**
- **FURUKAWA, Shoei**
  **Gifu-shi**
  **Gifu 501-1196 (JP)**
- **NAIKI, Mitsuru**
  **Kato-shi**
  **Hyogo 673-1461 (JP)**
- **OKADA, Tomoyuki**
  **Kato-shi**
  **Hyogo 673-1461 (JP)**
- **MATSUMOTO, Tomonori**
  **Kato-shi**
  **Hyogo 673-1461 (JP)**
- **SAWADA, Kazuyoshi**
  **Kato-shi**
  **Hyogo 673-1461 (JP)**

(74) Representative: **HOFFMANN EITLE**
**Patent- und Rechtsanwälte**
**Arabellastrasse 4**
**81925 München (DE)**

# (54) PROPHYLACTIC OR THERAPEUTIC DRUG FOR PERIPHERAL NEUROPATHY CAUSED BY ANTICANCER AGENT

(57) An object of the present invention is to provide a drug which is effective for prophylaxis or therapy of peripheral nerve disorder occurred as a side effect of the administration of anti-cancer agents.

The present invention is to provide a drug containing an ester of $C_{10}$ fatty acid as an active ingredient for prophylaxis or therapy of peripheral nerve disorder induced by administration of a drug containing at least one of anti-cancer agents. The drug of the present invention containing the compound as an active ingredient is highly useful as a drug for prophylaxis or therapy of peripheral nerve disorder induced by administration of anti-cancer agents.

[Fig. 1]

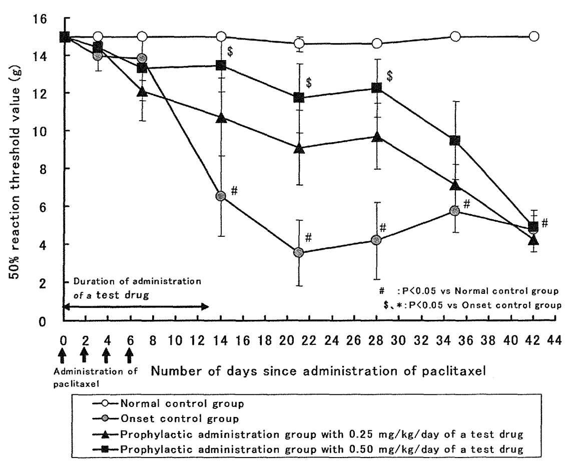


EP 2 671 578 A1

## Description

### [Technical Field]

The present invention relates to a novel pharmaceutical use of an ester of $C_{10}$ fatty acid and, more particularly, it relates to a drug containing an ester of $C_{10}$ fatty acid as an active ingredient for prophylaxis or therapy of peripheral nerve disorder induced by administration of a drug containing at least one of anti-cancer agents.

### [Background Art]

In current cancer (malignant tumor) treatment, surgery, irradiation or chemotherapy is used alone or in any combination thereof as required. Among them, anti-cancer agents (anti-malignant-tumor agents) used in the chemotherapy inherently have cytotoxicity or cell inhibition and damage not only the cancer (malignant tumor) cells but also human normal cells to cause side effects. Thus, it is important that the anti-cancer agents are administered to patients so as to prevent or reduce such side effects as far as possible and to provide sufficient anti-cancer (anti-malignant-tumor) effects.

Examples of the side effects caused by administration of anti-cancer agents include variously blood disorders, gastrointestinal disorders, nerve disorders, etc. and, in particular, acute or chronic nerve disorders have increased as a recent trend. This trend is considered to be caused by the following factors: frequent occurrence of nerve disorders as a main side effect of new anti-cancer agents providing remarkable anticancer effects, the effects of multiple drug therapy as recent main therapy, and an improving tendency of side effects such as blood disorders and gastrointestinal disorders. In this manner, no effective countermeasures against the nerve disorders, which are a main side effect caused by the current cancer chemotherapy, are available once the disorders have developed, due to the difficulty of nerve cell regeneration. Therefore, serious symptoms or irreversible disorders may be caused because of the difficulty of nerve cell regeneration. Accordingly, the nerve disorders that are the main side effect are an important therapeutic problem.

The nerve disorders caused by administration of anti-cancer agents are observed in, besides the central nervous system, the autonomic nervous system, and the peripheral nervous system, the sense organs such as the sense of taste. Among them, nerve disorders in the peripheral nervous system that occur in a comparatively high frequency to be problems are pains such as a stinging pain and burning pain, paresthesia such as numbness of limb extremities and a burning sensation, hyperesthesia such as hypersensitivity to cold stimuli, dysesthesia such as sensory loss, sensory paralysis and sense of discomfort, sensory ataxia, muscle weakness or the like. The lesions in the peripheral nervous system induced by administration of anti-cancer agents are considered mainly due to axonal degeneration, although in the present invention the anti-cancer agent inducing the peripheral nerve disorder may be any kind of them. Microtubules in the axon play an important role in maintaining the normal function of cells, for example, forming a spindle during cell division, and involving in placing the subcellular organelle and transporting substances. A taxane drug such as paclitaxel and docetaxel and a vinca alkaloid drug such as vincristine, vinblastine, vindesine and vinorelbine target the microtubules to inhibit the proliferation of cancer cells. Thus, it is considered that the microtubules in normal nerve cells are also commonly damaged to cause the nerve disorder. Furthermore, it is considered that platinum drugs such as oxaliplatin, carboplatin, cisplatin and nedaplatin directly damage nerve cells and consequently axonopathy is secondarily caused.

In spite of the above situation, the neurotoxicity of the anti-cancer agents has not been studied in detail and sufficient preventive and supportive methods for the peripheral nerve disorder which is a side effect caused by taxane anti-cancer drugs, etc. as above described have yet to be established. Therefore, at present, for relieving numbness symptoms, vitamin $B_{12}$ preparations such as mecobalamin and a Chinese herbal medicine, Gosha-jinki-gan, are used. For pains, an antidepressant (amitriptyline hydrochloride), an antiepileptic agent (carbamazepine), an antiarrhythmic agent (mexiletine hydrochloride), adrenocorticosteroid and the like are used. However, these therapies have limited effectiveness. Accordingly, stopping/reducing the administration of anti-cancer agent is the only reliable method for preventing the development of the peripheral nerve disorder (however, even after stopping the administration, the peripheral nerve disorder may continue or get worse). It would require the administration of anti-cancer agents for treatment of cancer, but a significant peripheral nerve disorder makes it difficult to continue the administration of important anti-cancer agents which are highly effective against cancer and it becomes a serious problem for treatment of cancer. In view of the above problems, prophylactic or therapeutic agents more effective against a peripheral nerve disorder induced by anti-cancer agents have been strongly required in clinical practices.

The present invention relates to a drug for prophylaxis or therapy of peripheral nerve disorder by anti-cancer agents containing an ester of fatty acid as an active ingredient. (In the present application, the term "peripheral nerve disorder" can be substituted with "neuropathy" which is a synonym. Further, any of the terms "prophylaxis" and "therapy" includes "improvement" or "alleviation" of peripheral nerve disorder by anti-cancer agents.) Patent Document 1 discloses that a $C_8$ fatty acid or $C_{10\sim12}$ fatty acids as well as ester thereof has/have a neurotrophic factor-like activity. However, the agent having a neurotrophic factor-like activity of Patent Document 1 is merely disclosed to such an effect that it is

useful as a prophylactic/therapeutic agent for neurodegenerative disease such as Alzheimer disease or Parkinson disease and mental disease such as depression or anxiety disorder (neurosis) and, unlike the present invention, there is no disclosure therein at all for its prophylactic or therapeutic action for peripheral nerve disorder resulted as a side effect of anti-cancer agents.

In addition, Non-Patent Document 1 reports that decenoic acid which is a component specific to royal jelly has a suppressive action for neurite retraction induced by cisplatin which is an anti-cancer agent. However, according to the content of the poster of the concerned societies, this decenoic acid is 10-hydroxyl-2-decenoic acid and is a compound having a different structure from the ester of $C_{10}$ fatty acid which is an active ingredient of the drug of the present invention in such a respect that the position 10 of its fatty acid chain is substituted with a hydroxyl group and that it is not an ester.

As mentioned hereinabove, it has not been known yet that an ester of $C_{10}$ fatty acid is effective for prophylaxis or therapy of peripheral nerve disorder by administration of anti-cancer agents.

**[Prior Art Documents]**

**[Patent Document]**

Patent Document 1: International Publication No. WO 2009/038110 **[Non-Patent Document]**

Non-Patent Document 1: Joint Conference of the 33rd Annual Meeting of the Molecular Biology Society of Japan with the 83rd General Meeting of the Japanese Biochemical Society. Subject: 4P-0977, General Subject (with Poster) "Action of decenoic acid on cisplatin-induced neurite retraction of nerve cell-like PC 12 cells" (Presented on December 10, 2010)

**[Summary of the Invention]**

**[Problems to be solved by the Invention]**

An object of the present invention is to provide a drug which is effective for prophylaxis or therapy of peripheral nerve disorder expressed as a side effect upon administration of anti-cancer agents.

**[Means for Solving the Problems]**

The present inventors have found as a result of the experiments to be hereinafter described that an ester of $C_{10}$ fatty acid shows an excellent prophylactic or therapeutic effect to peripheral nerve disorder by anti-cancer agents and accomplished the present invention. Thus, the present invention is as follows.

(1) A drug containing an ester of $C_{10}$ fatty acid as an active ingredient for prophylaxis or therapy of peripheral nerve disorder induced by administration of a drug containing at least one of anti-cancer agents.
(2) The drug according to (1), wherein the fatty acid in the ester of  fatty acid is decenoic acid.
(3) The drug according to (2), wherein the decenoic acid is 2-decenoic acid.
(4) The drug according to (3), wherein the 2-decenoic acid is trans-2-decenoic acid.
(5) The drug according to any of (1) to (4), wherein the ester in the ester of fatty acid is an alkyl ester.
(6) The drug according to any of (1) to (4), wherein the ester in the ester of fatty acid is an alkenyl ester.
(7) The drug according any of (1) to (4), wherein the ester in the ester of fatty acid is a cycloalkyl ester.
(8) The drug according to any of (1) to (7), wherein the anti-cancer agent is a microtubule inhibitor.
(9) The drug according to (8), wherein the microtubule inhibitor is a taxane drug.
(10) The drug according to (9), wherein the taxane drug is paclitaxel or docetaxel.
(11) The drug according to (9), wherein the taxane drug is paclitaxel.
(12) The drug according to (8), wherein the microtubule inhibitor is a vinca alkaloid drug.
(13) The drug according to (12), wherein the vinca alkaloid drug is vincristine.
(14) The drug according to any of (1) to (7), wherein the anti-cancer agent is a platinum drug.
(15) The drug according to (14), wherein the platinum drug is oxaliplatin or cisplatin.
(16) The drug according to any of (1) to (15), wherein the peripheral nerve disorder is acute or chronic pain, numbness, paresthesia, hyperesthesia or dysesthesia.

(17) The drug according to any of (1) to (16), wherein the drug is an injectable preparation.
(18) The drug according to any of (1) to (16), wherein the drug is an oral preparation.
(19) The drug according to (17) or (18), wherein the drug is a cyclodextrin inclusion complex.
(20) The drug according to any of (1) to (16), wherein the drug is an external preparation.
(21) The drug according to (20), wherein the external preparation is a patch preparation.
(22) The ester of $C_{10}$ fatty acid according to any of (1) to (7)for use in the prophylaxis or therapy of peripheral nerve disorder induced by administration of a drug containing at least one of anti-cancer agents.
(23) A method for prophylaxis or therapy of peripheral nerve disorder induced by administration of a drug containing at least one of anti-cancer agents, comprising administering the ester of $C_{10}$ fatty acid according to any of (1) to (7) in effective dose to a patient suffering from peripheral nerve disorder induced by administration of a drug containing at least one of anti-cancer agents.
(24) Use of the ester of $C_{10}$ fatty acid according to any of (1) to (7) in the manufacture of a drug for prophylaxis or therapy of peripheral nerve disorder induced by administration of a drug containing at least one of anti-cancer agents.

**[Advantages of the Invention]**

The ester of $C_{10}$ fatty acid in accordance with the drug of the present invention has such an excellent pharmacological action that it prevents or treats peripheral nerve disorder induced by administration of anti-cancer agents and is very highly useful.

**[Brief Description of the Drawings]**

[Fig. 1] Fig. 1 is a result where the effect of prophylactic administration of a drug of the present invention to hyperalgesia induced by administration of paclitaxel was tested.
[Fig. 2] Fig. 2 is a result where the effect of therapeutic administration of a drug of the present invention to hyperalgesia induced by administration of paclitaxel was tested.
[Fig. 3] Fig. 3 is a result where the effect of prophylactic administration of a drug of the present invention to hyperalgesia induced by administration of oxaliplatin was tested.
[Fig. 4] Fig. 4 is a result where the effect of therapeutic administration of a drug of the present invention to hyperalgesia induced by administration of oxaliplatin was tested.

**[Mode for Carrying Out the Invention]**

The present invention relates to a drug containing an ester of $C_{10}$ fatty acid as an active ingredient for prophylaxis or therapy of peripheral nerve disorder induced by administration of a drug containing at least one of anti-cancer agents.
The ester of fatty acid which is able to be utilized as an active ingredient of the drug of the present invention is an ester of fatty acid comprising a $C_{10}$ fatty acid and an alcohol, and the ester of fatty acid as such may be used solely or two or more thereof may be used in combination. Although the $C_{10}$ fatty acid may be any of decanoic acid (caprylic acid) which is a linear saturated fatty acid, decenoic acid which is a linear unsaturated fatty acid and geranic acid which is a branched unsaturated fatty acid, a preferred one is an unsaturated fatty acid having one double bond of carbons such as 2-decenoic acid, 3-decenoic acid or 9-decenoic acid, more preferred one is a trans compound thereof and the particularly preferred one is trans-2-decenoic acid.
On the other hand, with regard to the alcohol forming an ester moiety of the fatty acid ester in the drug of the present invention, there may be exemplified an alkyl alcohol, an alkenyl alcohol and a cycloalkyl alcohol.
Although there is no particular limitation for the alkyl alcohol, a preferred one is an alcohol having a linear or branched alkyl having 1 to 12 carbon(s) such as methyl, ethyl, propyl, isopropyl, butyl, isobutyl, sec-butyl, tert-butyl, pentyl, isopentyl, neopentyl, tert-pentyl, hexyl, isohexyl, heptyl, isoheptyl, octyl, isooctyl, nonyl, isononyl, decyl, isodecyl, undecyl, isoundecyl, dodecyl or isododecyl.
Although there is no particular limitation for the alkenyl alcohol, a preferred one is an alcohol having a linear or branched alkenyl having 2 to 12 carbons such as ethenyl, propenyl, isopropenyl, butenyl, isobutenyl, sec-butenyl, tert-butenyl, pentenyl, isopentenyl, neopentenyl, tert-pentenyl, hexenyl, isohexenyl, heptenyl, isoheptenyl, octenyl, isooctenyl, nonenyl, isononenyl, decenyl, isodecenyl, undecenyl, isoundecenyl, dodecenyl or isododecenyl and a more preferred one is an alcohol having a linear or branched alkenyl having 9 to 11 carbons such as nonenyl, isononenyl, decenyl, isodecenyl, undecenyl or isoundecenyl.
Although there is no particular limitation for the cycloalkyl alcohol, a preferred one is an alcohol having a cycloalkyl having 3 to 8 carbons such as cyclopropyl, cyclobutyl, cyclopentyl, cyclohexyl, cycloheptyl or cyclooctyl and

a more preferred one is an alcohol having a cycloalkyl having 5 or 6 carbons such as cyclopentyl or cyclohexyl.

The known compounds mentioned in Patent Document 1 or commercially available compounds can be used as the ester of $C_{10}$ fatty acid which is an active ingredient of a drug in the present invention. The ester of $C_{10}$ fatty acid can also be produced by known methods, for example, by subjecting a $C_{10}$ fatty acid and an alcohol to a dehydration-condensation. The dehydration-condensation reaction may adopt the conventionally known methods.

As a known condensation method, for example, a $C_{10}$ fatty acid may be made to react with an alcohol in the presence of an appropriate condensing agent (such as dicyclohexylcarbodiimide (DCC) or N-(3-dimethylaminopropyl)-N'-ethylcarbodiimide'HCl). The reaction may be usually carried out in a common solvent (such as dichloromethane). Usually, the using amount of the alcohol is 0.5 to 2 mol (preferably, 1 to 1.5 mol) to 1 mol of the $C_{10}$ fatty acid.

Alternatively, a $C_{10}$ fatty acid may be, for example, once converted to a carboxylic halide and then made to react with an alcohol in the presence or absence of a base. Conversion to the carboxylic halide may be carried out, for example, using a halogenating agent such as thionyl chloride, sulfyryl chloride, phosphorus trichloride, phosphorus pentachloride, oxalyl chloride or phosphoric acid trichloride. Examples of the base include triethylamine and pyridine. Usually, the using amount of the alcohol is 0.5 to 2 mol (preferably, 1 to 1.5 mol) to 1 mol of the $C_{10}$ fatty acid. When a base is used, the using amount of the base is usually about 1 to 5 mol to 1 mol of the $C_{10}$ fatty acid.

Examples of the compound produced as such are shown in Tables 1 and 2. When each compound is referred to hereinafter, the compound number mentioned in the tables is used.

[Table 1]

| Compound No. | Compound Name | Structural Formula |
|---|---|---|
| 1 | Decanoic acid methyl ester | 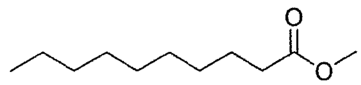 |
| 2 | Decanoic acid ethyl ester | 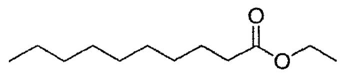 |
| 3 | Trans-2-decenoic acid methyl ester | 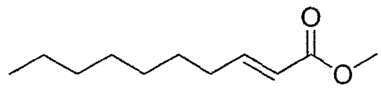 |
| 4 | Trans-2-decenoic acid ethyl ester | 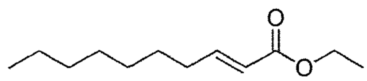 |
| 5 | Trans-2-decenoic acid propyl ester | 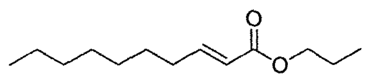 |
| 6 | Trans-2-decenoic acid isopropyl ester | 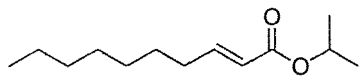 |
| 7 | Trans-2-decenoic acid butyl ester | 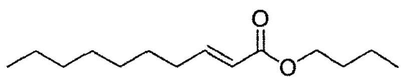 |
| 8 | Trans-2-decenoic acid *sec*-butyl ester | 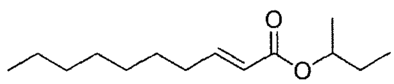 |
| 9 | Trans-2-decenoic acid isobutyl ester | 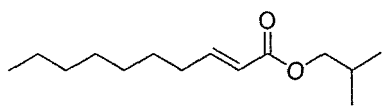 |
| 10 | Trans-2-decenoic acid *tert*-butyl ester | 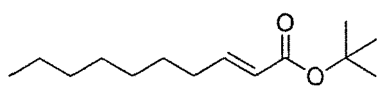 |
| 11 | Trans-2-decenoic acid hexyl ester | 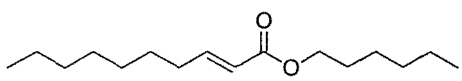 |
| 12 | Trans-2-decenoic acid heptyl ester | 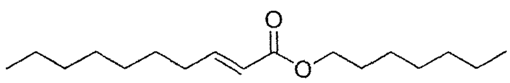 |

(continued)

| Compound No. | Compound Name | Structural Formula |
|---|---|---|
| **13** | Trans-2-decenoic acid octyl ester | 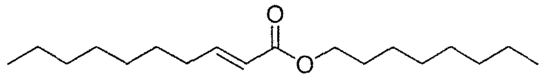 |
| **14** | Trans-2-decenoic acid decyl ester | 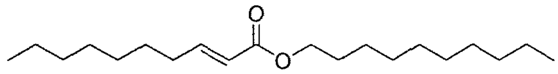 |
| **15** | Trans-2-decenoic acid undecyl ester | 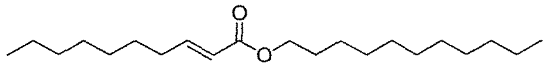 |

[Table 2]

| Compound No. | Compound Name | Structural Formula |
|---|---|---|
| **16** | Trans-2-decenoic acid trans-2-decenyl ester | 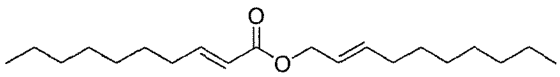 |
| **17** | Trans-2-decenoic acid trans-9-decenyl ester | 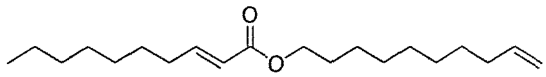 |
| **18** | Trans-2-decenoic acid cyclohexyl ester | 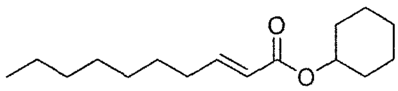 |
| **19** | Trans-3-decenoic acid methyl ester | 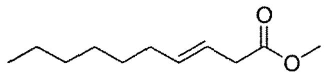 |
| **20** | Trans-3-decenoic acid ethyl ester | 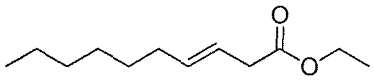 |
| **21** | Trans-9-decenoic acid methyl ester | 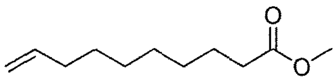 |
| **22** | Trans-9-decenoic acid ethyl ester | 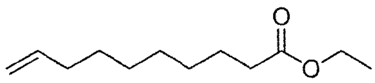 |
| **23** | Geranic acid ethyl ester | 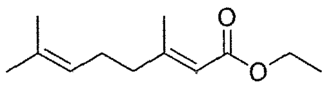 |

With regard to the compounds 8, 9, 12, 14 15 an 17 which have not been mentioned in the documents yet among the compounds mentioned in Tables 1 and 2, they were synthesized by means of the above-mentioned reaction using trans-2-decenoic acid and an appropriate alcohol corresponding to each ester moiety and were purified using silica gel chromatography or the like whereupon each of the compounds was produced as an oily substance.

A drug of the present invention contains an ester of $C_{10}$ fatty acid as an active ingredient, and is effective as a prophylaxis or therapy agent for a peripheral nerve disorder induced by administration of anti-cancer agents. One of the anti-cancer agents developing the peripheral nerve disorder is an anti-cancer agent that damages microtubules to induce the peripheral nerve disorder. Examples of such medicinal agent include taxane drug such as paclitaxel or docetaxel and vinca alkaloid drug such as vincristine, vinblastine, vindesine or vinorelbine. In addition, there is an anti -canceragent that damages nerve cells to cause axonopathy and then induces the peripheral nerve disorder. Examples of such agent include platinum drug such as oxaliplatin, carboplatin, cisplatin or nedaplatin.

Examples of the peripheral nerve disorder induced by these anti-cancer agents include pain such as a stinging pain and burning pain, numbness of limb extremities, paresthesia such as a burning sensation, hyperesthesia such as hypersensitivity to cold stimuli, dysesthesia such as sensory loss, sensory paralysis and sense of discomfort, sensory ataxia and muscle weakness. The peripheral nerve disorder induced by anti-cancer agents targeted for the prophylaxis

or therapy in the present invention includes a peripheral nerve disorder induced by monotherapy using one of anti-cancer agents as well as a peripheral nerve disorder induced by multiple drug therapy in which a plurality of medicinal agents having various action mechanisms is administered or by biochemical modulation in which a combination of medicinal agents and an administration method are designed such that the medicinal agents having various action mechanisms can provide the maximum effectiveness.

The ester of $C_{10}$ fatty acid of the present invention can be made into a pharmaceutical preparation in various dosage forms (such as oral, injectable and external preparations) by appropriately combining with an appropriate pharmaceutical carrier or diluent. The drug of the present invention may be also a combination drug in which the ester of $C_{10}$ fatty acid is combined with other pharmaceutically active ingredient(s). Further, the drug of the present invention may be made into a preparation as a cyclodextrin inclusion complex or the like. As a result, there may be the cases where enhancement of pharmacological activity, improvement in stability, prolonged action, easy handling, etc. can be achieved. An inclusion complex can be formed by, for example, mixing an ester of $C_{10}$ fatty acid with $\alpha$-, $\beta$- or $\gamma$-cyclodextrin whereby enhancement of pharmacological activity upon, for example, oral administration is noted.

When the drug of the present invention is made into an oral preparation, it is possible to make into tablet, powder, granule or capsule preparation by means of such a formulation where the ester of $C_{10}$ fatty acid is appropriately combined with an appropriate additive such as excipient, binder, disintegrator, lubricant, extender, wetting agent, buffer, preservative or flavoring. In making into an injectable preparation, it is possible to make into an injectable preparation by addition of stabilizer, preservative, isotonic agent or the like to a solution or suspension containing the ester of $C_{10}$ fatty acid. In making into an external preparation, it is possible to make into an external preparation such as patch preparation, gel preparation, ointment, cream preparation or the like. Thus, the ester of $C_{10}$ fatty acid is, for example, mixed with, melted in or emulsified in an appropriate base and, in the case of a patch preparation, the above is spread and applied onto a support. In the case of a patch preparation, a gel preparation or the like, it can be made, for example, into a composition using an organogelling agent. Incidentally, depending upon the dosage form of each external preparation, a commonly used preservative, antioxidant, flavoring agent, adhesive or the like may be appropriately selected and added to a formulation.

Adequate dose of the compound of the present invention may be appropriately increased or decreased by taking dose regimen, age, sex, symptom in a patient, etc. into consideration and, may be generally administered in an amount of from 1 to 1,000 mg or, preferably, 5 to 300 mg, for adult, at ounce or in several divided administrations per day.

**[Examples]**

Hereinafter, an example of results of a pharmacological test concerning novel pharmacological activity of the drug of the present invention, that is, a prophylactic or therapeutic effect to the peripheral nerve disorder induced by anti-cancer agents, is described. The present invention is not intended to be limited to the descriptions in Examples.

Pharmacological tests: Action to hyperalgesia of anti-cancer agent -induced peripheral nerve disorder rats

Effect of the drug of the present invention to peripheral nerve disorder induced by administration of an anti-cancer agent was investigated by means of an action of the drug of the present invention to hyperalgesia such as allodynia (pain induced by tactile stimulation which usually cause no pain) induced by administration of an anti-cancer agent to animals.

1. Effect of the repeated intraperitoneal administrations of the drug of the present invention to hyperalgesia caused by an anti-cancer agent

[1] Effect to hyperalgesia by the administration of paclitaxel

(1) Organization of groups of experimental animals

With regard to male SD rats of six weeks age used as the experimental animals, their 50% reaction threshold values to the mechanical stimulus (the measuring method will be mentioned later) and body weights were measured and then the following seven groups each comprising eight rats was organized.

- Normal control group
- Onset control group
- Prophylactic administration group with 0.25 mg/kg/day of a test drug
- Prophylactic administration group with 0.50 mg/kg/day of a test drug
- Therapeutic administration group with 0.25 mg/kg/day of a test drug

- Therapeutic administration group with 0.50 mg/kg/day of a test drug
- Therapeutic administration group with 30 mg/kg/day of gabapentin (positive control)

(2) Preparation of paclitaxel-induced peripheral nerve disorder rats

Taxol (registered trademark) injection 30 mg (manufactured by Bristol-Myers Squibb) containing 6 mg/mL of paclitaxel was used. To the rats of each of the above groups except the normal control group was intraperitoneally administered paclitaxel diluted to 2 mg/mL with saline in a dose of 2 mg/kg four times every other day whereupon the paclitaxel-induced peripheral nerve disorder rats were prepared. To the normal control group, an equivalent mixture of ethanol and Cremophor EL (registered trademark; manufactured by Sigma; a solvent for Taxisol (registered trademark) injection) diluted with saline to an extent of 3-fold was administered similarly.

(3) Administration of a test drug

To prophylactic administration groups of a test drug, the compound 4 in a dose of 0.25 mg/kg/day or 0.50 mg/kg/day was repeatedly administered intraperitoneally since immediately after initiation of administration of paclitaxel until 13 days thereafter. Meanwhile, to therapeutic administration groups of a test drug and to a therapeutic administration group of gabapentin, the compound 4 in a dose of 0.25 mg/kg/day or 0.50 mg/kg/day or gabapentin in a dose of 30 mg/kg/day, respectively was repeatedly administered intraperitoneally during the period of from 14 days to 42 days after initiation of the administration of paclitaxel.

(4) Result of measurement of the 50% reaction threshold values to the mechanical stimulus (von Frey test)

The seven groups of rats of the above (1) were placed in a transparent acrylic cage with a wire-meshed floor and habituated for about three minutes and the 50% reaction threshold values to the mechanical stimulus of right hind limb were measured after 3, 7, 14, 21, 28, 35 and 42 days from initiation of the administration of paclitaxel. Incidentally, during the period of administration of a test drug, its values were measured after 24 hours from the last administration of a test drug.
The measurement was conducted using von Frey filaments (manufactured by North Coast Medical Inc.) in accordance with the methods of Chaplan, et al. (Journal of Neuroscience Methods, vol. 53, no. 1, pages 55 to 63, 1994) and Dixon, et al. (Annual Review of Pharmacology and Toxicology, vol. 20, pages 441 to 462, 1980). In eight filaments [stimulus loads (g): 0.4, 0.6, 1.0, 2.0, 4.0, 6.0, 8.0 and 15.0], the test was started as from the filament of 2.0 g, the filament was vertically attached to the sole for 2 to 3 seconds with such a force that the filament was lightly bent and the case where the hind limb showed an escape reaction was called a positive reaction. The case where the rat escaped at the instance of removing the filament was also called positive. When the positive reaction was noted, stimulus was conducted similarly using a filament of one rank weaker while, when no reaction was noted, stimulus was conducted similarly using a filament of one rank stronger and the point when the reaction changed from negative to positive or from positive to negative was called the first two reactions. After that, stimulus was conducted for continuous four times by the same up-down method. A 50% reaction threshold value to the mechanical stimulus was measured using the reaction to the six stimuli in total and then (mean value) ± (standard error) for each group was calculated. Incidentally, when stimulus reached by that of 15.0 g without positive reaction or, when positive reaction continued to 0.4 g, then 15.0 g or 0.25 g was adopted as each threshold value, respectively.

The test for significant difference was performed using the test in two groups (Wilcoxon test or t-test) between the normal control group and the onset control group and between the onset control group and the gabapentin- administered group. Between the onset control group and the test drug-administered group, it was performed using the nonparametric or parametric Dunnett multiple comparison test. Analysis was conducted using SAS System Version 8.2 (SAS preclinical package Ver. 5.0, SAS Institute Japan) and, it was judged that $p<0.05$ is significantly different.

An example of the above test result is shown in Figs. 1 and 2. The 50% reaction threshold values to the mechanical stimulus of the onset control group to which paclitaxel was repeatedly administered for four times significantly lowered as compared with the normal control group during the period of from 14 days to 42 days after initiation of the administration of paclitaxel. In contrast, Fig. 1 is a graph showing the result where a test drug (the compound 4) was repeatedly subjected to prophylactic administration intraperitoneally during the period of immediately after administration of paclitaxel until 13 days thereafter. In a prophylactic administration group with 0.5 mg/kg/day of a test drug, significantly high 50% reaction threshold values as compared with the onset administration group were noted during the period of from 14 days to 28 days after initiation of the administration of paclitaxel. In the meanwhile, trans-2-decenoic acid which was not an ester was intraperitoneally administered in a dose of 0.50 mg/kg/day similarly but, in terms of 50% reaction threshold values to the mechanical stimulus, there is no difference from the onset control group whereby no prophylactic effect to hyperalgesia induced by administration of paclitaxel was noted.

On the other hand, Fig. 2 is a graph showing the result when a test drug (the compound 4) was repeatedly subjected to a therapeutic administration intraperitoneally during the period of from 14 days (where a 50% reaction threshold value significantly lowered) to 42 days after initiation of the administration of paclitaxel. In a therapeutic administration group with 0.25 mg/kg/day of a test drug during the period of from 21 days to 35 days after administration of paclitaxel and a therapeutic administration group with 0.5 mg/kg/day of a test drug after 28 days from administration of paclitaxel, significantly high 50% reaction threshold values as compared with an onset control group were noted.

[2] Effect to hyperalgesia by the administration of oxaliplatin

(1) Organization of groups of experimental animals

With regard to male SD rats of six weeks age used as the experimental animals, their 50% reaction threshold values to mechanical stimulus and body weights were measured and then the following seven groups each comprising 8 rats was organized.

- Normal control group
- Onset control group
- Prophylactic administration group with 0.25 mg/kg/day of a test drug
- Prophylactic administration group with 0.50 mg/kg/day of a test drug
- Therapeutic administration group with 0.25 mg/kg/day of a test drug
- Therapeutic administration group with 0.50 mg/kg/day of a test drug
- Therapeutic administration group with 10 mg/kg/day of pregabalin

(positive control)

(2) Preparation of oxaliplatin-induced peripheral nerve disorder rats

To the rats of each of the above groups except the normal control group was intraperitoneally administered an oxaliplatin solution dissolved in glucose injection to an extent of 4 mg/mL in a dose of 4 mg/kg twice weekly for four weeks being eight times in total whereupon the oxaliplatin-induced peripheral nerve disorder rats were prepared. To the normal control group, glucose injection was administered similarly.

(3) Administration of a test drug

To prophylactic administration groups of a test drug, a test drug (the compound 4) in a dose of 0.25 mg/kg/day or 0.50 mg/kg/day was repeatedly administered intraperitoneally since immediately after initiation of administration of oxaliplatin until 27 days thereafter. Meanwhile, to a therapeutic administration group of a test drug and to a therapeutic administration group of pregabalin, a test drug (the compound 4) in a dose of 0.25 mg/kg/day or 0.50 mg/kg/day was repeatedly administered intraperitoneally during the period of from 14 days to 27 days after initiation of the administration of oxaliplatin and, pregabalin was administered orally in repeated dose of 10 mg/kg/day during the same period.

(4) Result of measurement of 50% reaction threshold values to the mechanical stimulus (von Frey test)

A von Frey test was conducted in the same manner as in the above 1.[1](4) using the seven groups of rats mentioned in the above (1). 50% reaction threshold values in each group were measured before initiating the administration of oxaliplatin and after 7, 14, 21, 28, 33 and 40 days from the initiation of the administration. Mean value ± standard error for each group was calculated and the test for significant difference was conducted in the same manner as in the above 1.[1](4).

An example of the above test result is shown in Figs. 3 and 4. 50% reaction threshold values to the mechanical stimulus of the onset control group to which oxaliplatin was repeatedly administered significantly lowered as compared with the normal control group during the period of from 14 days to 40 days after initiation of the administration of oxaliplatin. In contrast, Fig. 3 is a graph showing the result where a test drug (the compound 4) was subjected to prophylactic administration intraperitoneally in repeated dose during the period of immediately after administration of oxaliplatin until 27 days thereafter. In a prophylactic administration group with 0.25 mg/kg/day of a test drug during the period of from 21 days to 28 days after administration of oxaliplatin and in a prophylactic administration group with 0.5 mg/kg/day of a test drug during the period of from 21 days to 33 days after administration of oxaliplatin, significantly high 50% reaction threshold values were noted as compared with the onset control group.

On the other hand, Fig. 4 is a graph showing the result when a test drug (the compound 4) was subjected to

therapeutic administration intraperitoneally in repeated dose during the period of from 14 days to 27 days after initiation of the administration of oxaliplatin. In a therapeutic administration group with 0.25 mg/kg/day of a test drug after 28 days from the administration of oxaliplatin and in a therapeutic administration group with 0.5 mg/kg/day of a test drug during the period of from 21 days to 33 days after the administration of oxaliplatin, significantly high 50% reaction threshold values as compared with an onset control group were noted.

Further, in each of the above pharmacological tests, the effect of suppressing the hyperalgesia of a test drug still continued even after completion of the repeated administration of a test drug. Furthermore, as shown in the result (Fig. 2 or 4) of a therapeutic effect of a test drug, the drug of the present invention showed an effect in such a low dose of 0.25 mg/kg/day or 0.50 mg/kg/day as compared with the dose (30 mg/kg/day) of gabapentin or the dose (10 mg/kg/day) of pregabalin.

2. Effect of the single intraperitoneal administration of the drug of the present invention to hyperalgesia induced by paclitaxel

(1) Preparation of paclitaxel-induced peripheral nerve disorder rats and administration of the test drug

Male SD rats in six weeks age (one group comprised six rats) were used as experimental animals and the paclitaxel-induced peripheral nerve disorder rats were prepared in the same manner as in the above 1.[1](2). The test drug was administered intraperitoneally in a single dose so as to make it 0.3 mg/kg in any of the cases during the period of 18 to 25 days or 20 to 27 days after initiation of administration of paclitaxel.

A von Frey test was conducted in the same manner as in the above 1.[1](4) using the rats mentioned in the above (1) and each 50% reaction threshold value was measured. With regard to the higher 50% reaction threshold value between the 50% reaction threshold values after 1 hour and 5 hours from administration of a test drug, a recovery rate (%) of the 50% reaction threshold value was calculated.

An example of this test result is shown in Table 3.

Recovery rate (%) of 50% reaction threshold value ={ [(50% reaction threshold value after 1 hour or 5 hours from administration of test drug) - (50% reaction threshold value before administration of test drug)] ÷ [(Normal threshold value) - (50% reaction threshold value before administration of test drug)]} × 100

[Table 3]

| Test drug | Recovery rate of 50% reaction threshold value (%) |
|---|---|
| Compound 2 | 22.9 |
| Compound 3 | 63.2 |
| Compound 4 | 48.5 |
| Compound 5 | 71.2 |
| Compound 6 | 10.2 |
| Compound 7 | 22.7 |
| Compound 10 | 50.0 |
| Compound 11 | 60.8 |
| Compound 12 | 21.3 |
| Compound 14 | 32.2 |
| Compound 16 | 94.4 |

(continued)

| Test drug | Recovery rate of 50% reaction threshold value (%) |
|---|---|
| Compound 17 | 54.0 |
| Compound 18 | 15.8 |
| Compound 20 | 14.7 |
| Compound 22 | 67.9 |

As will be apparent from Table 3, even when the drug of the present invention was intraperitoneally administered in a single dose, an excellent improving effect to hyperalgesia induced by administration of paclitaxel was noted.

3. Effect of the oral administration of the drug of the present invention to hyperalgesia by paclitaxel

(1) Preparation of an α-cyclodextrin (CD) inclusion complex of the compound of the present invention

A suspension (2.0 kg/L) 5 mL of the compound 4 was mixed with a 1% α-CD aqueous solution followed by stirring overnight to prepare an α-CD inclusion complex of the compound 4.

(2) Organization of groups of experimental animals

With regard to male SD rats of six weeks age used as the experimental animals, the rats were organized into the following six groups each comprising 6 rats.

- Oral administration group with 0.3 mg/kg of the test drug suspension
- Oral administration group with 3 mg/kg of the test drug suspension
- Oral administration group with an α-CD inclusion complex containing 0.3 mg/kg of the test drug
- Oral administration group with an α-CD inclusion complex containing 3 mg/kg of the test drug
- α-CD oral administration group
- Intraperitoneal administration group with 0.3 mg/kg of the test drug

(3) Preparation of paclitaxel-induced peripheral nerve disorder rats and administration of a test drug

Paclitaxel-induced peripheral nerve disorder rats were prepared in the same manner as in the above 1.[1](2). The compound 4 was used as a test drug and, after 39 days from the initiation of the administration of paclitaxel, it was orally administered in a single dose where the dose calculated as a test drug became 0.3 mg/kg or 3 mg/kg to oral administration groups with the test drug suspension or to oral administration groups with an α-CD inclusion complex containing the test drug while, to an intraperitoneal administration group with the test drug, it was intraperitoneally administered in a single dose of 0.3 mg/kg. Further, to an α-CD Oral administration group, an α-CD solution was orally administered in a single dose of 15 mg/kg.

(4) Result of measurement of 50% reaction threshold values to the mechanical stimulus (von Frey test)

A von Frey test was conducted in the same manner as in the above 1.[1](4) using the rats mentioned in the above (2) and 50% reaction threshold values in each group were measured before initiating the administration of a test drug and after 1, 5 and 24 hours. Further, AUC (area of the part surrounded by a curve and an abscissa axis (a time axis) in a graph showing the 50% reaction threshold value over time from administration of the test drug) was calculated and the relative ratio of each group was calculated where the AUC of the intraperitoneal administration group with 0.3 mg/kg of the test drug was adopted as 100.
An example of this test result is shown in Table 4.
[Table 4]

| Test group | 50% reaction threshold value | | AUC | |
|---|---|---|---|---|
| | Before administration | After 5 hours from adminitration | Measured value | Relative ratio |
| Oral administration group with 0.3 mg/kg of the test drug suspension | 4.57 | 6.00 | 31.83 | 43 |
| Oral administration group with 3 mg/kg of the test drug suspension | 4.57 | 8.66 | 54.99 | 74 |
| Oral administration group with an α-CD inclusion complex / 0.3 mg/kg of the test drug | 4.57 | 6.89 | 55.10 | 74 |
| Oral administration group with an α-CD inclusion complex / 3 mg/kg of the test drug | 4.53 | 9.98 | 85.21 | 114 |
| α-CD oral administration group | 4.57 | 5.74 | 19.63 | 26 |
| Intraperitoneal administration group with 0.3 mg/kg of the test drug | 4.58 | 8.57 | 74.59 | 100 |

As will be apparent from Table 4, even when the drug of the present invention was orally administered, an excellent improving effect to hyperalgesia induced by administration of paclitaxel was noted. It was further noted that, when the drug of the present invention was orally administered after making into an α-CD inclusion complex, a far better effect was available.

**[Industrial Applicability]**

The drug of the present invention has an excellent prophylactic or therapeutic effect to hyperalgesia caused by the mechanical stimulus resulted in the allodynia animal models prepared by administration of paclitaxel or oxaliplatin which is an anti-cancer agent. Accordingly, the drug of the present invention is highly useful as a drug for prophylaxis or therapy of peripheral nerve disorder in humans and animals including paresthesiasuch as numbness of limb extremities and hyperalgesia such as pain caused by anti-cancer agents.

## Claims

1. A drug containing an ester of $C_{10}$ fatty acid as an active ingredient for prophylaxis or therapy of peripheral nerve disorder induced by administration of a drug containing at least one of anti-cancer agents.

2. The drug according to claim 1, wherein the fatty acid in the ester of fatty acid is decenoic acid.

3. The drug according to claim 2, wherein the decenoic acid is 2-decenoic acid.

4. The drug according to claim 3, wherein the 2-decenoic acid is trans-2-decenoic acid.

5. The drug according to any of claims 1 to 4, wherein the ester in the ester of fatty acid is an alkyl ester.

6. The drug according to any of claims 1 to 4, wherein the ester in the ester of fatty acid is an alkenyl ester.

7. The drug according any of claims 1 to 4, wherein the ester in the ester of fatty acid is a cycloalkyl ester.

8. The drug according to any of claims 1 to 7, wherein the anti-cancer agent is a microtubule inhibitor.

9. The drug according to claim 8, wherein the microtubule inhibitor is a taxane drug.

10. The drug according to claim 9, wherein the taxane drug is paclitaxel or docetaxel.

11. The drug according to claim 9, wherein the taxane drug is paclitaxel.

12. The drug according to claim 8, wherein the microtubule inhibitor is a vinca alkaloid drug.

13. The drug according to claim 12, wherein the vinca alkaloid drug is vincristine.

14. The drug according to any of claims 1 to 7, wherein the anti-cancer agent is a platinum drug.

15. The drug according to claim 14, wherein the platinum drug is oxaliplatin or cisplatin.

16. The drug according to any of claims 1 to 15, wherein the peripheral nerve disorder is acute or chronic pain, numbness, paresthesia, hyperesthesia or dysesthesia.

17. The drug according to any of claims 1 to 16, wherein the drug is an injectable preparation.

18. The drug according to any of claims 1 to 16, wherein the drug is an oral preparation.

19. The drug according to claim 17 or 18, wherein the drug is a cyclodextrin inclusion complex.

20. The drug according to any of claims 1 to 16, wherein the drug is an external preparation.

21. The drug according to claim 20, wherein the external preparation is a patch preparation.

[Fig. 1]

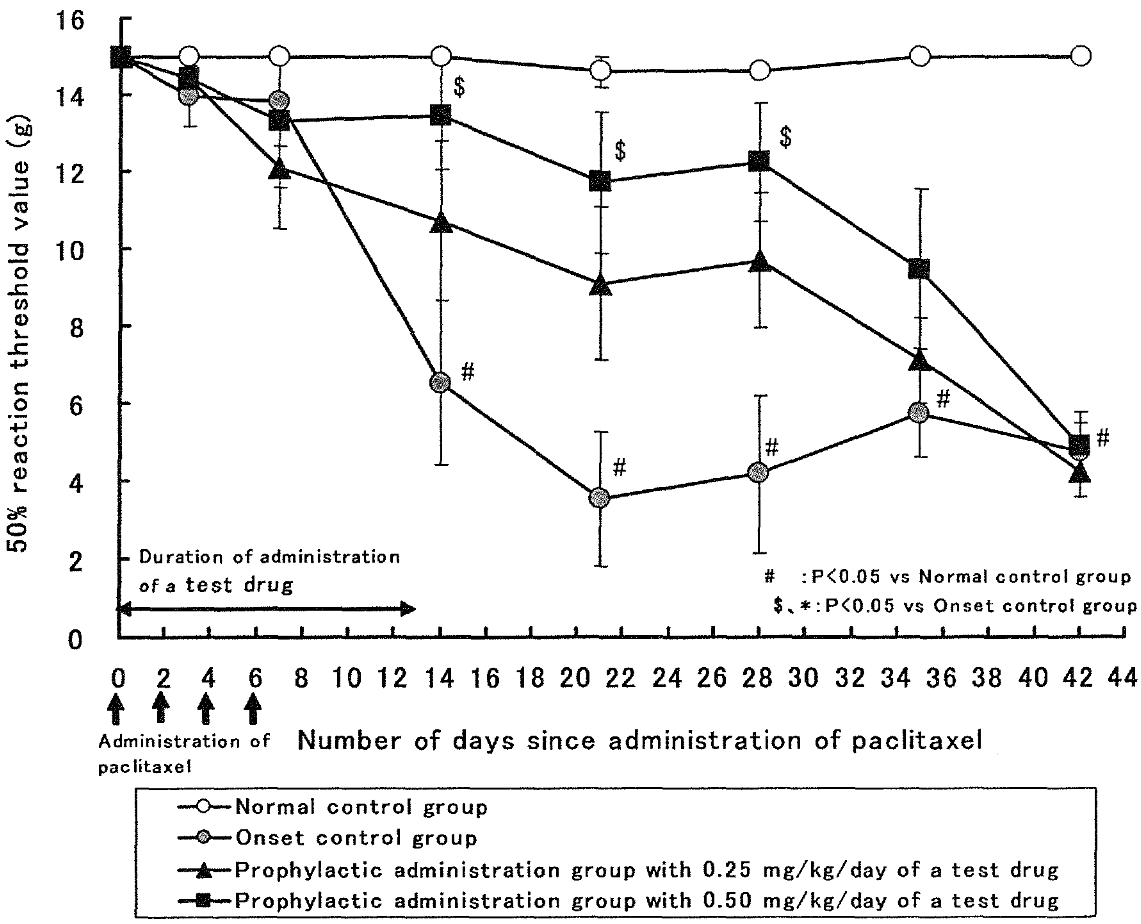

16
14
12
10
8
6
4
2
0
50% reaction threshold value (g)
$
$
$
#
#
#
#
#
Duration of administration of a test drug
# :P<0.05 vs Normal control group
$、*:P<0.05 vs Onset control group
0 2 4 6 8 10 12 14 16 18 20 22 24 26 28 30 32 34 36 38 40 42 44
Administration of paclitaxel
Number of days since administration of paclitaxel
Normal control group
Onset control group
Prophylactic administration group with 0.25 mg/kg/day of a test drug
Prophylactic administration group with 0.50 mg/kg/day of a test drug

[Fig. 2]

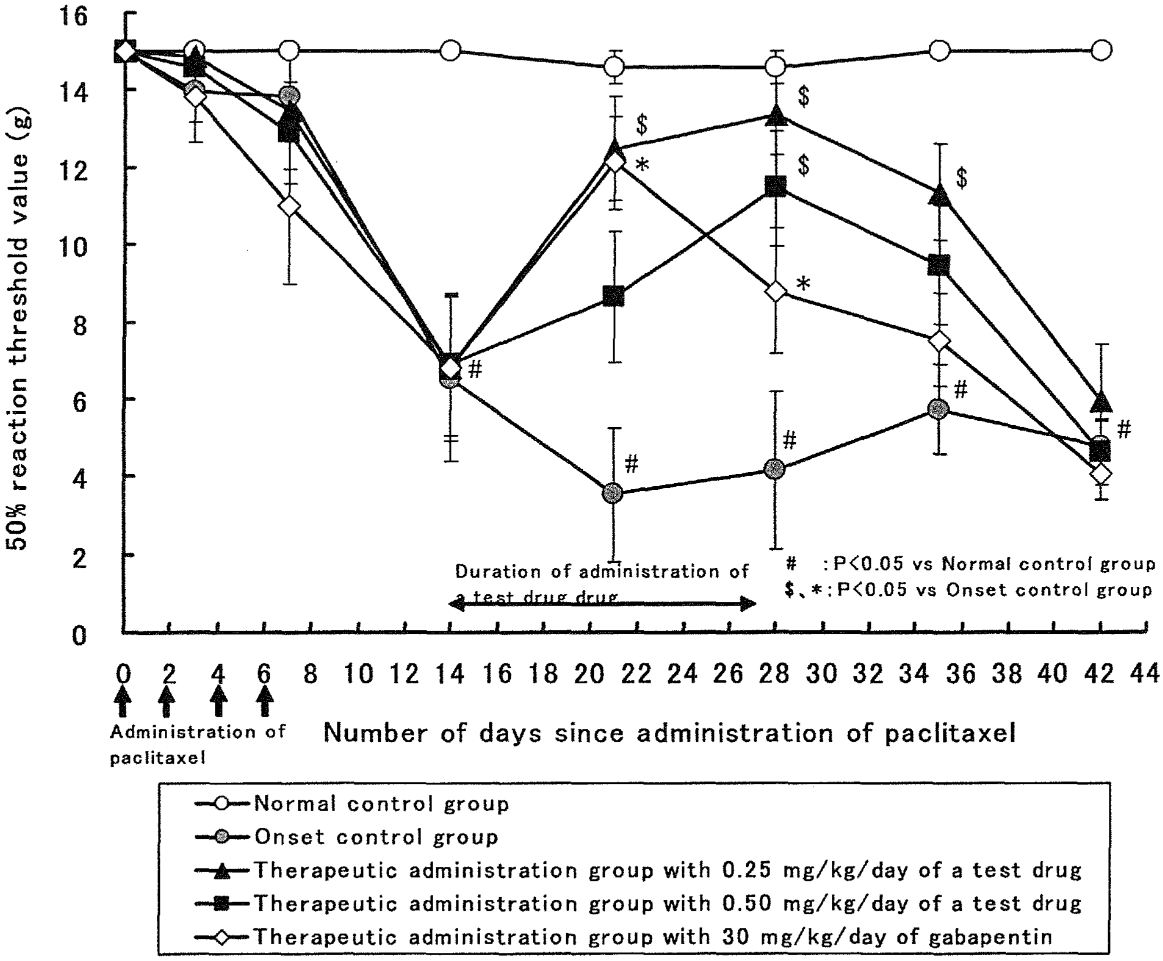
16
14
12
10
8
6
4
2
0
50% reaction threshold value (g)
$
$
$
$
*
*
#
#
#
#
#
Duration of administration of a test drug drug
# : P<0.05 vs Normal control group
$、*: P<0.05 vs Onset control group
0 2 4 6 8 10 12 14 16 18 20 22 24 26 28 30 32 34 36 38 40 42 44
Administration of paclitaxel
Number of days since administration of paclitaxel
Normal control group
Onset control group
Therapeutic administration group with 0.25 mg/kg/day of a test drug
Therapeutic administration group with 0.50 mg/kg/day of a test drug
Therapeutic administration group with 30 mg/kg/day of gabapentin

[Fig. 3]

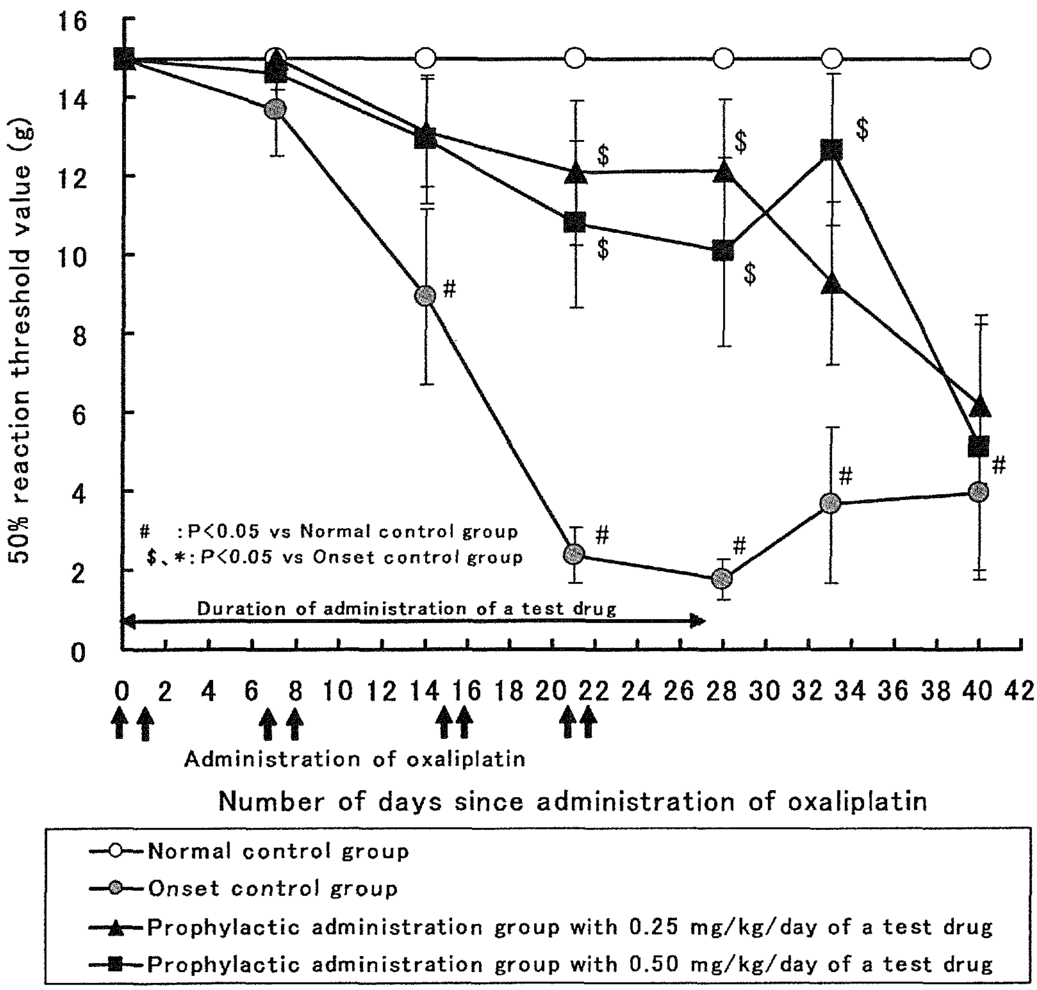
50% reaction threshold value (g)
16
14
12
10
8
6
4
2
0
# :P<0.05 vs Normal control group
$、*: P<0.05 vs Onset control group
Duration of administration of a test drug
0 2 4 6 8 10 12 14 16 18 20 22 24 26 28 30 32 34 36 38 40 42
Administration of oxaliplatin
Number of days since administration of oxaliplatin
Normal control group
Onset control group
Prophylactic administration group with 0.25 mg/kg/day of a test drug
Prophylactic administration group with 0.50 mg/kg/day of a test drug

[Fig. 4]

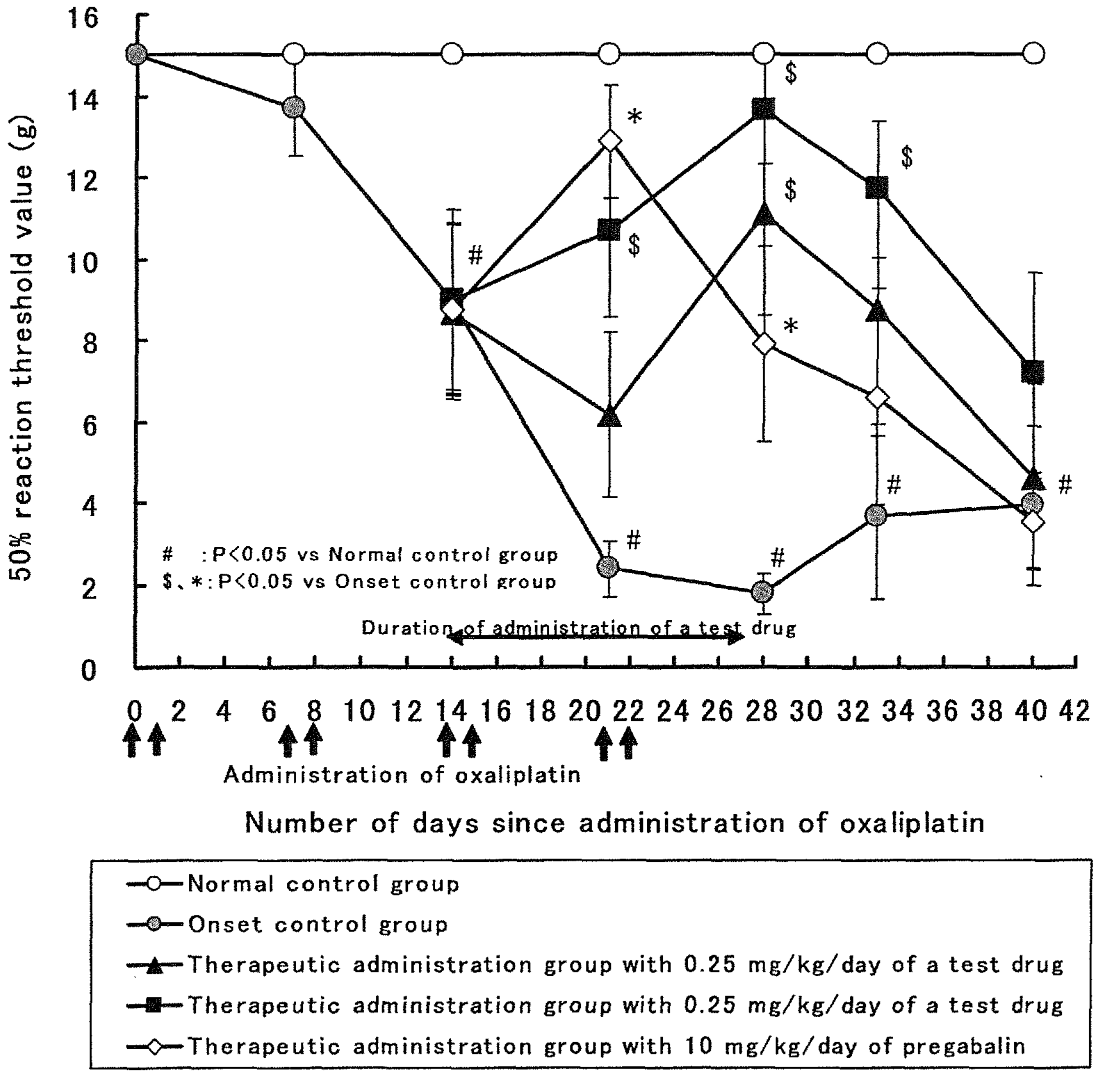

50% reaction threshold value (g)
16
14
12
10
8
6
4
2
0
# : P<0.05 vs Normal control group
$, *: P<0.05 vs Onset control group
Duration of administration of a test drug
0 2 4 6 8 10 12 14 16 18 20 22 24 26 28 30 32 34 36 38 40 42
Administration of oxaliplatin
Number of days since administration of oxaliplatin
Normal control group
Onset control group
Therapeutic administration group with 0.25 mg/kg/day of a test drug
Therapeutic administration group with 0.25 mg/kg/day of a test drug
Therapeutic administration group with 10 mg/kg/day of pregabalin

**INTERNATIONAL SEARCH REPORT**

International application No.
PCT/JP2012/051938

A. CLASSIFICATION OF SUBJECT MATTER
*A61K31/201*(2006.01)i, *A61P25/02*(2006.01)i

According to International Patent Classification (IPC) or to both national classification and IPC

B. FIELDS SEARCHED

Minimum documentation searched (classification system followed by classification symbols)
A61K31/201, A61P25/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | WO 2009/038110 A1 (INST NAGOYA IND SCIENCE RES), 26 March 2009 (26.03.2009), entire text & EP 2204172 A1 & US 2010/0204498 A1 | 1-21 |
| A | WO 2009/028605 A1 (UNIV KYUSHU NAT UNIV CORP.), 05 March 2009 (05.03.2009), entire text & EP 2191836 A1 & US 2011/0111051 A1 | 1-21 |
| A | JP 2010-106019 A (National University Corporation Kanazawa University), 13 May 2010 (13.05.2010), entire text (Family: none) | 1-21 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 February, 2012 (15.02.12) | 28 February, 2012 (28.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009038110 A


### Non-patent literature cited in the description


- Action of decenoic acid on cisplatin-induced neurite retraction of nerve cell-like PC 12 cells. *Joint Conference of the 33rd Annual Meeting of the Molecular Biology Society of Japan with the 83rd General Meeting of the Japanese Biochemical Society,* 10 December 2010
- **CHAPLAN et al.** *Journal of Neuroscience Methods,* 1994, vol. 53 (1), 55-63
- **DIXON et al.** *Annual Review of Pharmacology and Toxicology,* 1980, vol. 20, 441-462